# EUROPEAN PATENT APPLICATION

(11) **EP 3 769 743 A1**
(43) Date of publication of application: **27.01.2021**
(21) Application number: 20180790.6
(22) Date of filing: 18.06.2020
(51) Int. Cl.: A61H 35/00

(54) **LEG CARE APPARATUS**

(30) Priority: 24.07.2019 KR 20190089667; 24.07.2019 KR 20190089668; 24.07.2019 KR 20190089733
(71) Applicant: LG Electronics Inc., Yeongdeungpo-gu Seoul 07336 (KR)
(72) Inventor: YOO, Hyunsun, 08592 Seoul (KR); CHUN, Jaehung, 08592 Seoul (KR); KIM, Joogyeom, 08592 Seoul (KR)
(74) Representative: Vossius & Partner Patentanwälte Rechtsanwälte mbB

(57) **Abstract**

A leg care apparatus includes a main body configured to provide an action space in which a leg is accommodated, a bottom module which is placed on a bottom surface of the main body and in which components for a foot bath are accommodated, and a circulation air suction fan configured to exhaust air within the action space to the outside of the action space and blow the air exhausted to the outside of the action space again to the inside of the action space.

## Description

The present application claims priority under 35 U.S.C. 119 and 35 U.S.C. 365 to Korean Patent Application Nos. 10-2019-0089667, 10-2019-0089668, and 10-2019-0089733, filed on July 24, 2019, which is hereby incorporated by reference in its entirety.

The present disclosure relates to a leg care apparatus.

Foot bath is an action where user's feet are soaked in hot water for a predetermined time.

The foot bath is an action where heat is applied to the user's feet. In detail, the foot bath is known to be effective in improving various ailments such as blood circulation improvement, body temperature rise, improvement in feeling cold, improvement in sleep disorders, waste discharge, ingrown toenail prevention, plantar fasciitis improvement, stress relief, skin care, and the like by using heat transferred indirectly to the human body through the feet.

A device that provides hot water up to a height of the vicinity of an ankle to allow the user to soak their feet is widely known as a foot bath device that is capable of performing a foot bath. The foot bath device using water has limitations in that heat loss is large, the device is difficult to handle, and its use is troublesome because the feet have to be conduction-heated indirectly by heating water.

To solve this limitations, a foot bath device in which a heating element is provided inside a control space, and the foot bath is performed by using radiant heating using the heating element is being introduced. A foot bath machine using radiant heating is disclosed in Korean Patent registration No. 10-1145430.

The above-described device has the following limitations. First, there is inconvenience in that a user control panel is complicated to operate. Second, there is a limitation in that user's safety is threatened because a heating element is used. Third, there is a limitation in that the storage and movement of the device are difficult. Fourth, there is a limitation in that the device is frequently damaged due to having no rigidity. Fifth, it is troublesome to use because the control space is blocked by a plate. Sixth, there is a limitation in that only fomentation using the radiant heat is enabled in a radiant heating manner. Seventh, moisture and waste products retained in the foot bath space may decompose to cause bad smell.

Embodiments provide a leg care apparatus that includes a foot bath device to care a leg.

Embodiments also provide a leg care apparatus which is conveniently operated and used by a user.

Embodiments also provide a leg care apparatus in which a temperature control state is safely applied to a user's leg.

Embodiments also provide a leg care apparatus that is conveniently moved and stored.

Embodiments also provide a leg care apparatus that is prevented from being damaged due to rigidity and easy to be handled by a user.

Embodiments also provide a leg care apparatus that is capable of enjoying foot bath in various manners.

Embodiments also provide a leg care apparatus that is capable of removing odor generated in a space in which a leg is accommodated.

In one embodiment, a leg care apparatus includes: a main body configured to provide an action space in which a leg is accommodated; a bottom module which is placed on a bottom surface of the main body and in which components for foot bath are accommodated; and a circulation air suction fan configured to exhaust air within the action space to the outside of the action space and blow the air exhausted to the outside of the action space again to the inside of the action space. Therefore, the circulation passage inside and outside the action space may be provided to improve heat loss, and the internal air of the action space is reduced from moving upward, which affects a user.

An external air suction fan configured to introduce external air outside the action space into the action space may be provided to allow the external air to flow therein.

The leg care apparatus may further include a foot thermoelectric module provided in the bottom module and having one surface to correspond to one surface within the action space so that foot bath is performed by using heat or cold air in a conductive manner.

The leg care apparatus may further include a bottom heat exchange fan provided in the bottom module to heat-exchange with the other surface of the foot thermoelectric module, thereby smoothly coping with the large capacity thermoelectric module.

A suction passage guide disposed inside the bottom module may divide an inner space of the bottom module into a suction space through which the external air flows by the external air suction fan and a heat exchange space through which the external air flows by the bottom heat exchange fan. Accordingly, a space for heat exchange with the external air introduced into the action space may be separated so that the space is used intensively.

The discharge air of the external air suction fan and the discharge air of the circulating air suction fan may be mixed and introduced into the action space. Accordingly, the internal air of the action space may be created in various modes.

The external air suction fan may suction the external air from both sides of the bottom module and may suction more air through a narrow space.

The leg care apparatus may further include a blower corresponding to a rear surface of the action space, wherein the blower may include: a fan; and a wind direction controller provided at a discharge-side of the fan. Accordingly, since a medium for the foot bath is applied to a large area from the front to the rear, satisfaction with the function of the foot bath may be high.

Since the wind direction control device is configured to control a wind direction vertically, the foot bath for the entire leg lengthily placed in the vertical direction may be performed.

Left and right air blowers may be provided to perform the foot bath on both the left and right legs.

The leg care apparatus may further include an odor removing device configured to remove odor particles from the air discharged from the circulation air suction fan. Accordingly, the foot care apparatus may be used more cleanly by removing odor particles from inside the action space which is in an atmosphere of high humidity and intrinsic matter.

A blower may be further provided to blow air into the action space, and odor particles in the air blown by the blower may be introduced into the action space. An odor removing device may be disposed in a passage between a discharge-side of the circulation air suction fan and a suction-side of the blower. Accordingly, the odor particles floating in the air may not be discharged to the outside causing unpleasant smell to the user and be removed by the odor removing device on the circulation passage.

The odor removing device may include: a catalyst member; and a light emitting element configured to emit light to the catalyst member. Accordingly, the odor particles may be more quickly removed by using photocatalytic reaction.

The fragrance kit which provides fragrance to the passage of the discharge-side of the circulation air suction fan may be further provided to provide aroma fragrance in the air from which the odor particles are removed, thereby improving user's satisfaction.

In another embodiment, a leg care apparatus includes: a main body configured to provide an action space in which a leg is accommodated; a bottom module which is placed on a bottom surface of the main body and in which components for foot bath are accommodated; and an external air suction fan configured to blow air outside the auction space into the action space. Accordingly, the external air may be blown to the inside of the action space to perform the foot bath in the more pleasant atmosphere.

The leg care apparatus may further include a circulation air suction fan configured to exhaust air within the action space to the outside of the action space and blow the air exhausted to the outside of the action space again to the inside of the action space. Accordingly, the air in the action space may be prevented from being permanently exhausted to the outside. As a result, the medium for various foot baths may be recirculated to save energy.

At least one of the circulation air suction fan and the external air suction fan may have a flow rate that is adjustable. Accordingly, since an amount of external inflow air introduced from the outside of the action space and an amount of circulation air reintroduced after being exhausted into the action space are adjustable, types of foot bath may be more variously provided.

A wind volume flowing by the circulation air suction fan and a wind volume flowing by the external air suction fan may be different from each other. According to this technique, if the air volume is large when compared to the air volume flowing by the external air suction fan, air that is naturally discharged to the outside during the progress of the foot bath may be supplemented. On the contrary, when the air volume is larger than that flowing by the circulation air suction fan, it may be used for changing the air in the action space, drying the action space, and the like.

According to the present disclosure, the passage structure may be separated smoothly in a narrow space.

The at least two fans may include: a fan configured to blow air to the action space; and a fan configured to perform heat exchange between components. Accordingly, the space utilization may be improved.

The details of one or more embodiments are set forth in the accompanying drawings and the description below. Other features will be apparent from the description and drawings, and from the claims.

### BRIEF DESCRIPTION OF THE DRAWINGS

FIGS. 1 and 2 are front perspective views of a leg care apparatus according to an embodiment, wherein FIG. 1 illustrates a state in which the leg care apparatus is stored, and FIG. 2 illustrates a state in which the leg care apparatus is operated.
FIG. 3 is a schematic cross-sectional view taken along line A-A' of FIG. 2.
FIG. 4 is an exploded side view of the entire leg care apparatus according to an embodiment.
FIG. 5 is an exploded side view of a main body.
FIG. 6 is an exploded side view of an upper module.
FIG. 7 is an exploded side view of a side module.
FIG. 8 is an exploded side view of a bottom module.
FIG. 9 is a cross-sectional view that cuts vertically an approximately central portion of a lower portion of the leg care apparatus in a front and rear direction.
FIG. 10 is a cross-sectional view that cuts horizontally a lower portion of a bottom module of the leg care apparatus.
FIG. 11 is a schematic view illustrating the inside of an action space.
FIG. 12 is a view illustrating an air flow within the action space.
FIG. 13 is a view for explaining a circulation process of circulation air returning to the action space after being discharged from the action space.
FIG. 14 is a configuration view for explaining an example of a blower.
FIG. 15 is a view of a leg care apparatus according to another embodiment.
FIG. 16 is a view illustrating an example of an odor removing device.

Hereinafter, exemplary embodiments will be described in detail with reference to the accompanying drawings. The invention may, however, be embodied in many different forms and should not be construed as being limited to the embodiments set forth herein, and a person of ordinary skill in the art, who understands the spirit of the present invention, may readily implement other embodiments included within the scope of the same concept by adding, changing, deleting, and adding components. Thus, it should be understood that they are also included within the scope of the present invention.

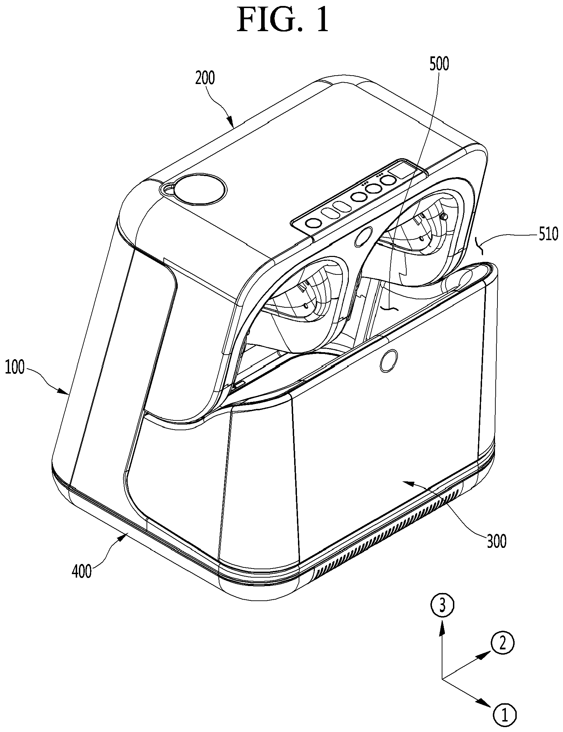

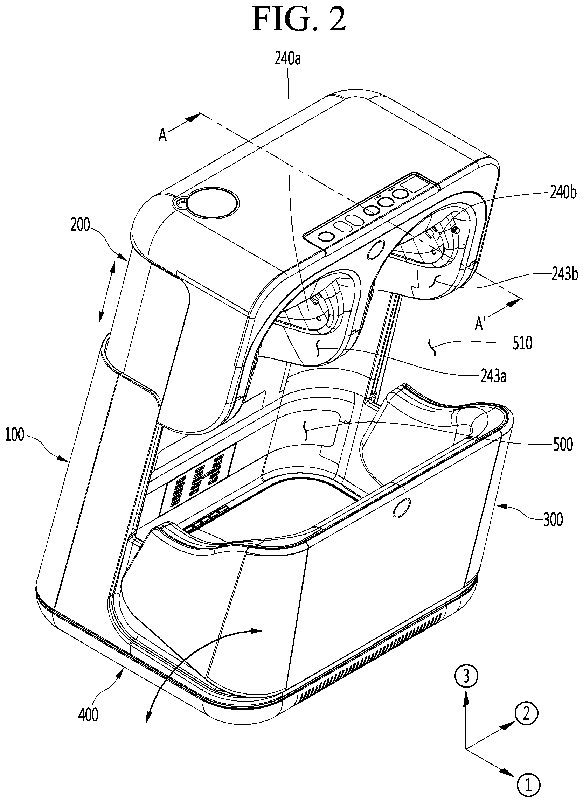

FIGS. 1 and 2 are front perspective views of a leg care apparatus according to an embodiment. That is, FIG. 1 illustrates a state in which the leg care apparatus is stored, and FIG. 2 illustrates a state in which the leg care apparatus is operated. Here, the storage state may mean a state in which the leg care apparatus has the smallest size or is not in use. The operation state may mean a state in which the leg care apparatus is expanded so that a user may insert their leg or a state in which the leg care apparatus is moved for use.

In the description of the drawings, a direction in which the user accesses the leg care apparatus indicates a front side. When based on each axis shown in the figures, the front and rear direction is expressed as ①, and the direction in which the user accesses the leg care apparatus indicates the front side. A left and right direction is expressed as ② and indicates a left and right direction of the front side with respect to the user. An upward and downward direction is expressed as ③ and indicates an upward and downward direction of the front side with respect to the user.

In the leg care apparatus according to an embodiment, in order to allow the user's leg to be inserted, an inlet may increase in size, and an inner action space may increase in volume. After the user's leg is inserted, the inlet may decrease in size to be suitable for the user's body, and the action space may decrease in volume to be suitable for the user's leg. Since the action space and the inlet are adjusted to be suitable for a body size of the user, particularly, a size and length of the leg, a thermal effect acting on the leg may be largely and quickly applied, and energy consumption may be saved.

According to an embodiment, the leg may be cared for by applying hot or cold air and/or pressure to a leg portion including portions of knees, calves, and thighs together with the feet.

In the following description, the meaning of the foot bath not only means foot bath using water pressure and heat applied in the water, but also applying heat, cold air, and pressure to a leg portion including portions of feet, knees, calves, and thighs.

Referring to FIGS. 1 and 2, a leg care apparatus 10 according to an embodiment includes a main body 100, an upper module 200 connected to an upper portion of the main body 100 to largely open an upper space of the leg care apparatus 10, a side module 300 connected to a front portion of the main body 100 to largely open an inner space of the leg care apparatus 10, and a bottom module 400 connected to a lower portion of the main body 100 to accommodate components that are required for operation of the leg care apparatus 10.

An action space 500 is provided in an inner space inside an inner surface of each of the main body 100, the upper module 200, the side module 300, and the bottom module 400. The action space 500 is a space for applying hot or cold air to a user's leg through at least one manner of conduction, convection, or radiation. An inlet 510 through which the user's leg is inserted and withdrawn is provided in front of the action space 500. Since at least one of the hot or cold air is applied to the user's leg in at least one manner of the conduction, convection, or radiation, the user may have a foot bath in a manner selected from various manners that are desired by the user.

The upper module 200 may perform a vertical elevation operation.

When the upper module 200 is moved upward, the inlet 510 is largely opened so that the user may conveniently insert their leg into the action space 500. After the user inserts their leg into the action space 500, the upper module 200 may be moved downward. The upper module 200 may be moved downward until a portion of the user's leg touches or the action space 500 is constructed in a shape desired by the user. The upper module 200 may define at least a portion of a top surface of the action space.

The upper module 200 is provided to be slid vertically in the embodiment, but is not limited thereto. For example, the upper module 200 may be opened through a rotation operation or moved to a position desired by the user.

Knee care parts 240a and 240b, each of which having a recessed shape, may be provided at both left and right side at the front of the upper module 200. Inner surfaces of the knee care parts may be provided with knee placing parts 243a and 243b. Each of the knee placing parts is a portion that contacts the user's knee. The knee placing part may include a light emitting element and a pad. The light emitting element and the pad may apply at least one of heat or pressure to care the knee, thereby performing blood flow improvement, muscle stimulation, and pain improvement.

The knee care part 240 cares the knee by applying at least one of the heat or the pressure. The action space 500 cares the user's leg through conduction, convection, and radiation of the hot or cold air. According to an embodiment, the leg care apparatus may improve user's satisfaction by performing a suitable action for each location of the leg. Particularly, the action space 500 may function as a foot bath machine by performing a function of the foot bath, and the knee care part 240 may function as a knee massager. The leg care apparatus according to embodiment may perform at least the functions of the foot bath machine and the knee massager.

The side module 300 may perform the rotation operation forward and backward.

When the side module 300 rotates forward, the inlet 510 may be opened so that the user may conveniently insert their leg into the action space 500. After the user inserts the leg into the action space, the side module 300 may rotate backward. The side module 300 may rotate backwards until a portion of the user's leg touches, or the action space 500 is constructed in a shape desired by the user. The side module 300 may provide at least a portion of a front surface of the action space.

The side module 300 rotates backward and forward in this embodiment, but the embodiment is not limited thereto. For example, the side module 300 may be slid to be opened or adjusted to a position desired by the user.

As described above, in the leg care apparatus 10 according to an embodiment, the upper module 200 and the side module 300 are contracted when not in use. Accordingly, the leg care apparatus 10 may be easily stored, moved, and handled in a state of being contracted in volume.

The upper module 200 and the side module 300 may be operable with respect to the main body 100. As a result, the action space may increase or decrease in volume. Thus, the functions such as the convenient handling, the foot bath that is suitable for the user, the leg contact, and the like may be performed. The upper module 200 and the side module 300 may perform the action of adjusting the size and shape of the action space. Thus, the upper module 200 and the side module 300 may be referred to as action space adjustment modules.

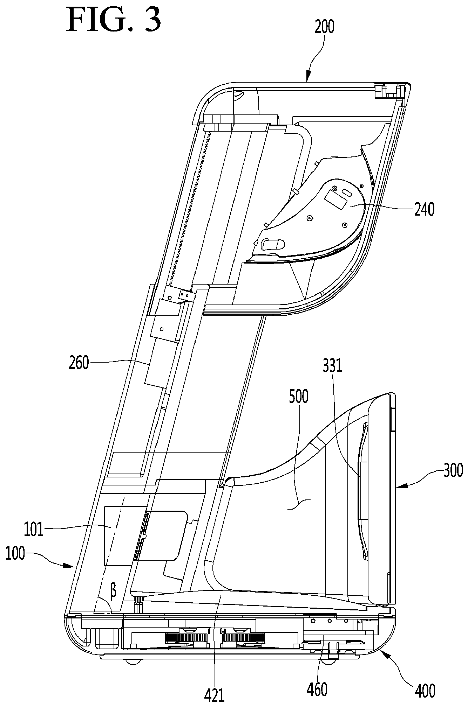

FIG. 3 is a schematic cross-sectional view taken along line A-A' of FIG. 2.

A schematic configuration and operation of the leg care apparatus according to an embodiment will be described with reference to FIG. 3. The main body 100 extends upward from a rear portion of the bottom module 400, and an upward extending angle is inclined forward at a predetermined angle β. Here, the inclined angle may be less than about 90 degrees as an acute angle. Since the main body 100 is inclined forward to extend, the user may not need to bend the knee excessively while inserting their leg into the action space 500 or while using the leg care apparatus.

Patients that need to care their leg by using the leg care apparatus may suffer from orthopedic diseases such as knee arthritis. The main body 100 may be provided to be inclined forward so that the action space 500 corresponds to a large bent angle of the user's leg without the patients having to excessively bend the knee. For example, the user may use the leg care apparatus even if the knee is not bent more than about 90 degrees.

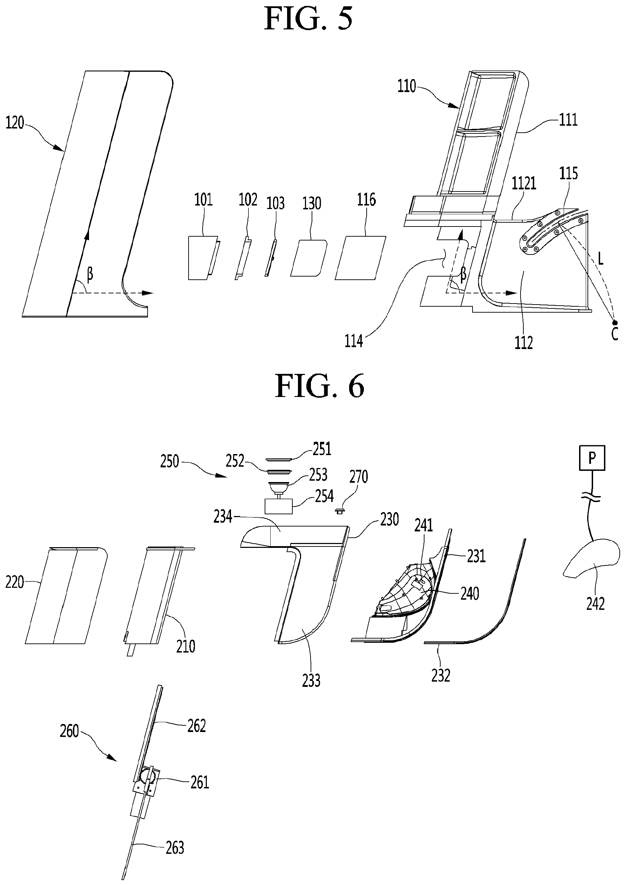

Since a main vertical extension part (see reference numeral 111 of FIG. 5) of the main body 100 is provided to be inclined forward, other components related thereto may also be provided to be inclined.

The upper module 200 is provided on an upper portion of the main body 100. A vertical opening device 260 may be inserted into a contact part between the upper module 200 and the main body 100. The vertical opening device 260 may include a driving motor and a gear train and may move the upper module 200 upward or downward with respect to the main body 100.

The upper module 200 being moved upward may be when the inlet 510 is opened so that the user's leg is inserted into the action space 500. Alternatively, the upper module 200 may be moved upward even when the user withdraws their leg from the action space 500. The upper module 200 being moved downward may be when the inlet 510 decreases in size, or the action space is contracted after the user inserts their leg.

The knee care part 240 may be disposed on a front portion of the upper module 200 to care the user's knee.

A blower 101 may be provided below the main body 100. The blower 101 may provide hot air into the action space 500. The hot air of the blower 101 may be heated by a heating wire provided in the blower 101. The blower 101 may perform an action for forced convection of air heated by an external separate heating device.

The bottom module 400 may be disposed on a bottom part of the leg care apparatus to support the entire apparatus at a lower side. A foot contact pad 421 may be disposed on a top surface of the bottom module 400. A sole of the foot may contact the foot contact pad 421. The foot contact pad 421 may perform a foot bath function by conducting a temperature atmosphere controlled by an external force to the user's foot.

A front and rear opening device 460 may be disposed on a front portion of the bottom module 400. The front and rear opening device 460 may include a motor and a gear train and may be inserted into a contact part between the bottom module 400 and the side module 300. The front and rear opening device 460 may move the side module 300 forward and backward with respect to the main body 100.

A calf contact pad 331 may be disposed on an inner surface of the side module 300. A user's calf may contact the calf contact pad 331. The calf contact pad 331 may perform a foot bath function by conducting a temperature atmosphere controlled by an external force to the user's calf.

The side module 300 rotating forward may be when the inlet 510 is opened so that the user's leg is inserted into the action space 500. Alternatively, the side module 300 may be moved forward even when the user withdraws their leg from the action space 500. The side module 300 being moved backward may be when the inlet 510 decreases in size, and the action space is contracted, or the calf contacts the calf contact pad 331 after the user inserts their leg.

When the upper module 200 and the side module 300 open the inlet 510, the upper module 200 may start the opening thereof first, and then, the side module 300 may be opened. This is done because the upper module 200 performs the sliding operation, while the side module 300 rotates, and thus, if the side module 300 rotates forward first, the side module 300 may interfere with the upper module 200.

When the upper module 200 and the side module 300 close the inlet 510, the upper module 200 and the side module 300 may be operated in reverse. For example, the side module 300 may be closed first at a predetermined angle, and then the side module 300 may be closed. Since the respective modules are operated in this order, the interference between the modules may be prevented.

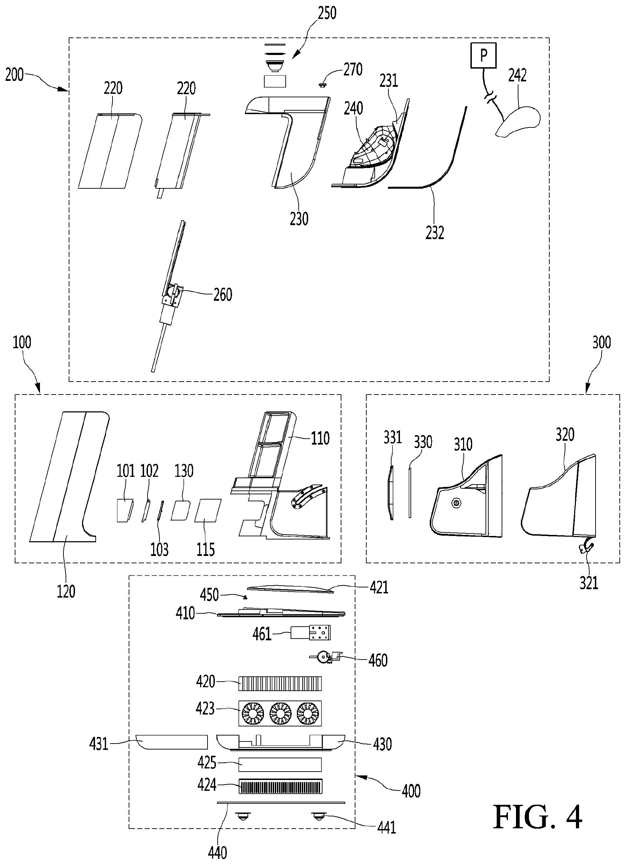

FIG. 4 is an exploded side view of the entire leg care apparatus according to an embodiment. Constituents of each module of the leg care apparatus according to the embodiment will be described with reference to FIG. 4.

First, the main body 100 is provided with a main frame 110 and a main body outer cover 120 provided on a rear surface of the main frame 110. A predetermined empty space may be provided between the main frame 110 and the main body outer cover 120, and components required for operating the leg care apparatus may be accommodated in the empty space.

The main body 100 may include a blower 101, a fragrance case 102 for accommodating a fragrance kit 103, and an atomizer 130. In addition, a heat generator, a radiant heater, and a cooler may be further provided.

The blower 101 is a device for generating a forced air current in the action space 500. The fragrance kit 103 may be provided as a device that provides fragrance into the action space 500 or remove a smell from the action space 500.

The atomizer 130 may supply mist to the inside of the action space 500 in at least one manner selected from ultrasonic spraying and heating spraying of water. A case in which both types of mist providing manners are installed may also be included in the embodiment.

The upper module 200 includes an upper frame 210 to which a portion of a movable member of the vertical opening device 260 is fixed to be elevated with respect to the main frame 100. An upper inner cover 230 and an upper outer cover 220 may be provided at an inner side and an outer side of the upper frame 210, respectively, to define an outer appearance of the upper module 200.

The knee care part 240, and a knee care seating panel 232 for mounting the knee care part 240 may be provided in front of the upper inner cover 230.

The side module 300 may include a side frame 310 and a side outer cover 320 provided in front of the side frame 310.

A calf thermoelectric module 330 and the calf contact pad 331 may be provided on an inner surface of the side module 300. A thermoelectric element may be provided in the calf thermoelectric module 330 to supply cold and hot air as desired by the user.

The bottom module 400 includes a bottom frame 410, a bottom housing 430 accommodating an outer edge of the bottom frame 410, and a bottom plate 440 that opens and closes a lower portion of the bottom frame 410.

A bottom supporter 441 provided as a wheel or the like is provided on a bottom surface of the bottom plate 440 so that the user easily move the leg care apparatus.

The foot thermoelectric module 420 and the foot contact pad 421 that transfers the cold and hot air of the foot thermoelectric module 420 to the user's foot in a conduction manner may be provided inside the bottom housing 430. The foot thermoelectric module 420 and the foot contact pad 421 may contact each other to transfer heat. The heat exchange fan 423, the grill 424, and the filter 425 may be further provided as constituents for the hot or cold air that is exhausted from the foot thermoelectric module 420 to the outside.

The front and rear opening device 460 may be accommodated in the bottom housing 430 so that the side module 430 rotates. The front and rear opening device 460 may be provided with a rotation driving part 461 including at least a motor and a link driving part 462 including a power transmission part such as a gear.

The bottom housing 430 is provided with a light emitting element 450 that is exposed upward so that heat is irradiated to the user's foot. In this case, the light emitting element may irradiate infrared rays. The light emitting element 450 may be provided as an ultraviolet lamp to sterilize and disinfect the action space 500.

A water tray 431 that stores water to be wasted and through which the stored wasted water is removed as necessary may be further provided at one side of the bottom housing 430. Water condensed after being atomized from the atomizer 130 to perform a predetermined function may be dropped into and stored in the water tray 431.

Hereinafter, each constituent of the leg care apparatus will be described in more detail.

FIG. 5 is an exploded side view of the main body. A configuration and operation of the main body will be described in more detail with reference to FIGS. 4 and 5.

Referring to FIGS. 4 and 5, the main body 100 may be largely divided into a main frame 110 that defines an overall shape of the leg care apparatus and supports a load of the leg care apparatus, and a main body outer cover 120 providing a predetermined space for accommodating components between the main body outer cover 120 and the main frame 110 and disposed behind the main frame 110.

At least a portion of the upper module 200 may be inserted into an interval between the main frame 110 and the main body outer cover 120, and thus, the upper module 200 may be vertically movable in a state of being guided to the main body 100. For this, the vertical opening device 260 may be accommodated in the interval between the main frame 110 and the main body outer cover 120.

The main frame 110 may be provided with a main front and rear extension part 112 extending forward and backward from a lower portion thereof and a main vertical extension part 111 extending upward from a rear portion of the main front and rear extension part 112. The main vertical extension part 111 may extend forward in a state of being inclined at a predetermined angle β with respect to the main front and rear extension part 112. The predetermined angle may be an acute angle. Thus, the user may insert their leg into the action space 500 in a more comfortable posture and use the leg care apparatus.

The main front and rear extension part 112 may be provided to close both sides of the lower portion of the action space 500. Thus, the forced air current within the action space 500 may not be lost through both side surfaces of the action space 500.

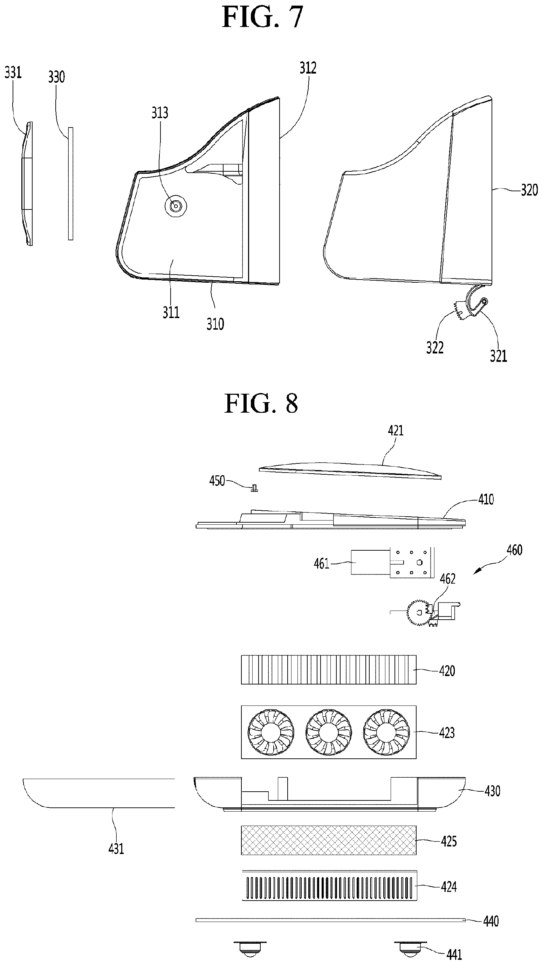

A guide slot 115 that guides the rotation of the side module 300 may be provided in the main front and rear extension part 112. The guide slot 115 may be provided to open the main front and rear extension part 112 in a curved shape and also be provided to define a groove having a curved shape in the main front and rear extension part 112. A protrusion (see reference numeral 313 of FIG. 7) of the side module 300 may be placed to be guided within the guide slot 115.

To guide the protrusion 313, the guide slot 115 may be provided as a curve having a geometric center with respect to a predetermined rotation center point C. The guide slot 115 may be provided in a curved shape having a predetermined length L as a curvature radius at the rotational center point C. The rotation center point C may be one point of a movement support part (see reference numeral 321 of FIG. 7) of the side module 300.

The main front and rear extension part 112 is completely closed except for a region of the guide slot 115. The guide slot 115 may be completely covered by the side frame 310 of the side module 300. This is the same as in a case in which the side module 330 completely rotates forward to be opened. Thus, both spaces of the action space 500 may be completely covered, and the forced air, which is artificially manipulated, in the action space 500 may leak to the outside.

For this, the side frame 310 may accommodate the main front and rear extension part 112 therein. Also, a flow blocking film 1121 that blocks the air leakage of the action space may extend up to an upper end of the main front and rear extension part 112. The flow blocking film 1121 may block the action space 500 even when the side module 300 is opened to cover the inside of the action space 500 from the outside.

An operation of the flow blocking film 1121 may be seen in FIG. 2. FIG. 2 illustrates a state in which the flow blocking film 1121 is exposed to the outside of a side portion side surface part 311 to cover the action space 500 in a state in which the side module 300 is opened.

Referring to FIG. 5, a main rear surface part 113 having a rear opening 114 may be provided on a rear surface of the main front and rear extension part 112. Components that provide various atmospheres required for the operation of the action space 500 may be mounted at a rear side of the main rear surface part 113. An operation medium that provides an atmosphere of the action space 500, such as air, light, and mist may pass through the rear surface opening 114.

The components that are placed at the rear side of the main rear surface part 113 may include the blower 101 that performs a blowing operation, the fragrance kit 103 that cleanly maintains the action space, the fragrance case 102 in which the fragrance kit 103 is accommodated, and the atomizer 130 that provides mist. Alternatively, other components may be further provided for a smooth operation of the action space 500.

The blower 101 may suction air from at least one of the inside or the outside of the action space 500 to supply the air to the action space 500. Here, the air supplied into the action space 500 may be artificially controlled in temperature. To control the temperature, the blower 101 may be provided with a separate temperature controller that is exemplified as the heat generator and the cooler.

The blower 101 may suction air within the action space 500 to apply a predetermined artificial operation to the suctioned air, thereby supplying the air into the action space 500. This may be understood as an air circulation inside the action space 500. Accordingly, energy efficiency may be improved by reducing the operation medium disposed to the outside.

An example of the fragrance kit 103 may include perfume and a photocatalyst smell decomposition device. The perfume may be a component that supplies an artificially good smell. The photocatalyst smell decomposition device is a member that is exemplified as titanium oxide and may be a device for decomposing smell particles by a catalytic action using action light such as ultraviolet light.

The atomizer 130 is a device for supplying mist. When the atomizer 130 is operated in the ultrasonic spraying manner, the mist may be supplied to the inside of the action space 500 without being hot, the legs may be cared while being cool, and the inside of the action space 500 may be cool through latent heat and the like. When the atomizer 130 is operated in the heating spray manner, the mist may be supplied to the inside of the action space 500 in a hot state, the leg may be warmed while taking the foot bath, and the inside of the action space 500 may be warmed.

The atomizer 130 may be provided with an ultrasonic spray device and a heating spray device. In this case, since the leg care apparatus is used in more various manners, the user's satisfaction may be improved.

The mist supplied from the atomizer 130 may perform a predetermined action in the action space 500.

For example, the high-temperature mist contacting the user's leg may transfer heat to the user's leg in a conduction manner. The high-temperature mist may be condensed on a surface of the user's leg and then heated by external hot air so that the foot bath is performed by continuously transferring heat to the user's leg in the conduction manner. For another example, the mist condensed on the user's leg may be evaporated to take on the cold fomentation on the user's leg.

The rear surface opening 114 may be closed by the main rear cover 116. The main rear cover 116 may be provided in a shape in which a hole is processed to allow the operation medium to pass therethrough.

The main body outer cover 120 may be provided in a shape that is inclined forward toward an upper side, like the main vertical extension part 111.

FIG. 6 is an exploded side view of the upper module 200. A configuration and operation of the upper module 200 will be described in more detail with reference to FIGS. 4 and 6.

Referring to FIGS. 4 and 6, the upper module 200 may be moved upward or downward with respect to the main body 100 by the vertical opening device 260. Here, the upper frame 210 may be a component constituting a frame of the upper module 200 and extend to be obliquely inclined forward like the main frame 110.

The vertical opening device 260 may include an elevation driving part 261 including at least a motor, an upper rail 262 extending upward from the elevation driving part 261, and a lower rail 263 extending downward from the elevation driving part 261. The upper rail 262 may be coupled directly or indirectly to the upper frame 210. The lower rail 263 may be coupled directly or indirectly to the main frame 110. At least one of the upper rail 262 or the lower rail 263 may be moved to allow the upper module 200 to ascend or descend.

The upper inner cover 230 and the upper outer cover 220 may be respectively coupled to front and rear portions of the upper frame 210 to define an outer appearance of the leg care apparatus. When the upper frame 210 is moved, the upper inner cover 230 and the upper outer cover 220 may be moved together.

The upper inner cover 230 may include an upper side surface part 233 extending vertically and inclined forward and an upper portion top surface part 234 extending backward from an upper end of the upper side surface part 233 and providing an upper end surface of the leg care apparatus.

The upper portion top surface part 234 may be a surface that is mainly observed when the user uses the leg care apparatus, and thus may be used variously. For example, the upper portion top surface part 234 may be provided with a water supply device 250 that supplies water used in the atomizer 130 and a display 270 that allows the user to control the leg care apparatus.

The water supply device 250 may include a water supply frame 254 in which the supplied water is primarily stored, a water supply supporter 253 that injects water into the water supply frame 254, and a water supply seating panel 252 that supports a water supply cover 251. The user may conveniently supply water by using the water supply device 250.

The display 270 may display information that is necessary for the operation of the leg care apparatus. Manipulation information that is necessary for controlling the leg care apparatus may be inputted by using the display 270. The display 270 may be provided as a touch panel.

The knee care part 240 may be disposed on a front portion of the upper module 200 to care the user's knee. The knee care part 240 may be provided to the knee care frame 231. To allow the knee care frame 231 to be coupled to the upper inner cover 230, a knee care seating panel 232 may be further provided.

The knee care part 240 may include at least one light emitting element 241 that irradiates infrared rays to the knee, at least one massage pad 242 that presses a spaced peripheral portion of patella, and a pump P that controls air pressure to the inside of the massage pad 242. The massage pad 242 may be applied in other methods such as spring pressure control rather than the air pressure control.

The knee care part 240 may include a knee placing part 243. The at least one light emitting element 241 and the at least one massage pad 242 are placed at positions of an inner region of the knee placing part 243, respectively. The knee placing part 243 may be a structure in which a material such as a soft cushion is filled and may apply an overall pressure to the user's knee to care the knee comfortably. According to the knee placing part 243, the action due to the massage pad 242 may be more improved.

Unlike the action space 500, as described above, the knee care part 240 performs an action such as pain relief of the knee by applying pressure and heat.

FIG. 7 is an exploded side view of the side module 300. A configuration and operation of the side module 300 will be described in more detail with reference to FIGS. 4 and 7.

Referring to FIGS. 4 and 7, the side module 300 rotates backward and forward so that the user may conveniently use the leg care apparatus.

The side module 300 may include a side frame 310 connected to the main frame 110 and a side outer cover 320 provided in front of the side frame 310.

The side frame 310 may include a side portion front surface part 312 and a side portion side surface part 311 extending backward from both sides of the side portion front surface part 312. The side portion side surface part 311 may be provided as two left and right walls, and the main front and rear extension part 112 may be inserted into an inner spaces of the two walls.

A protrusion 313 may be provided inside the side portion side surface part 311, and the protrusion 313 may be guided by the guide slot 115 (see FIG. 5). The positions at which the protrusion 313 and the guide slot 115 are provided may be opposite to each other. However, for stable operation, it is preferable that the protrusion 313 is provided on the side module 300, the guide slot 115 is provided on the main body 100.

A movement support part 321 supporting the rotation operation of the side module 300 may be provided on a lower portion of a front end of the side outer cover 320. The movement support part 321 may be hung and supported at any point of the bottom module 400 or the main body 100. The movement support part 321 may act as a center point of relative rotation with respect to the main body 100 of the side module 300.

A movement contact part 322 is provided at an adjacent position of the movement support part 321 to receive driving force of the link driving part 462 (see FIG. 8). For example, the link driving part 462 and the movement contact part 322 may be engaged with each other to receive the driving force of the rotation driving part 461.

An interaction between the main body 100 and the side module 300 may be performed by the rotation operation through the transmission of the driving force of the front and rear opening device 460 and the guiding action of the protrusion 313 and the guide slot 115.

The rotation driving force may be transmitted from the bottom module 400 to the side module 300 by the action connected to the rotation driving part 461, the link driving part 462, and the movement contact part 322 in time series. Here, the side module 300 may rotate in a state of being supported by the movement support part 321.

When the side module 300 is rotated by the rotation driving force, the protrusion 313 of the side module 300 may be guided by being placed inside the guide slot 115. The side module 300 may rotate at a curvature radius by a correct rotation center by the mutual guiding action of the guide slot 115 and the protrusion 313.

A calf thermoelectric module 330 and the calf contact pad 331 may be provided on an inner surface of the side module 300. A thermoelectric element may be provided in the calf thermoelectric module 330 to supply cold and hot air as desired by the user. Accordingly, the foot bath function for the calf portion of the user may be performed.

When the calf thermoelectric module 330 has a large heat load, a separate heat exchange fan may be installed like the foot thermoelectric module 420.

FIG. 8 is an exploded side view of the bottom module 400. A configuration and operation of the bottom module 400 will be described in more detail with reference to FIGS. 4 and 8.

Referring to FIGS. 4 and 8, a plurality of components for the foot bath may be provided in the bottom module 400. The bottom module 400 includes a bottom frame 410, a bottom housing 430 accommodating an outer edge of the bottom frame 410, and a bottom plate 440 that opens and closes a lower portion of the bottom frame 410.

The foot thermoelectric module 420 and the foot contact pad 421 that transfers the cold and hot air of the foot thermoelectric module 420 to the user's foot in a conduction manner may be provided inside the bottom housing 430. The foot thermoelectric module 420 and the foot contact pad 421 may contact each other to transfer heat. The foot contact pad 421 may contact the sole of the user, and the hot or cold air may be transferred to the sole of the foot to perform the foot bath function.

The foot contact pad 421 may be made of a metal having high thermal conductivity, for example, copper or stainless steel so as to uniformly transfer heat to the entire sole of the foot. This may be equally applied to the calf contact pad 331.

When water having a predetermined level is accumulated in the bottom housing 430, the foot contact pad 421 may heat the accumulated water to perform the foot bath function for the foot.

The heat exchange fan 423, the grill 424, and the filter 425 may be further provided as constituents for the hot or cold air that is exhausted from the foot thermoelectric module 420 to the outside. High energy may be supplied to the foot thermoelectric module 420 to supply a large amount of hot or cold air when compared to the calf thermoelectric module 330. Heat generated in and exhausted from the thermoelectric module 420 may be smoothly discharged to the outside by the heat exchange fan 423.

To allow the air circulated to the heat exchange fan 423 to perform a cooling operation without any problem, the grill 424 and the filter 425 may be provided. The air in which foreign substances are filtered by the filter 425 may be supplied to the blower 101 and supplied to the action space 500. In this case, cleaner air may be supplied to the action space 500 to improve the user's satisfaction.

The front and rear opening devices 460 are accommodated in the bottom housing 430 to allow the side module 430 to rotate as described above. A large portion of the front and rear opening device 460 is accommodated in the bottom module 400, but is not limited thereto. For example, the front and rear opening device 460 may be provided in the main body 100.

The bottom housing 430 is provided with a light emitting element 450 that is exposed upward so that heat is irradiated to the user's foot. The light emitting element 450 may perform various functions such as sterilization, ultraviolet light for photocatalytic decomposition, infrared rays, and the like depending on the emitted light.

The water tray 431 that stores wasted water to be discharged and wastes may be further provided at one side of the bottom housing 430. In the water tray 431, water condensed after being atomized by the atomizer 130 may be dropped into and stored. The water tray 431 is provided as a component that is slid to be separated to the outside. A valve may be provided in a passage through which water flows into the water tray 431 to prevent the water from leaking during the foot bath.

A bottom supporter 441 provided as a wheel or the like is provided on a bottom surface of the bottom plate 440 so that the user easily move the leg care apparatus. The bottom supporter 441 is provided as a rotatable wheel so that the user conveniently moves and uses the leg care apparatus in various directions. In the case of the elderly, the advantages of the above-described moving device may be largely utilized.

In the leg care apparatus according to the embodiment, the conductive heat may be transferred to the leg through the contact pad. In the leg care apparatus according to the embodiment, hot air may be blown to the leg through the blower 101 to transfer the convection heat. Alternatively, the blower 101 may blow cold air to the leg, but may be a representative example of blowing hot air for the foot bath represented by heating.

A purpose to the blowing of air into the action space 500 by the blower 101 is to transfer air that is a medium of the foot bath to the user's leg. In achieving such a purpose, it is necessary to further consider the following matters.

First, when the medium blown into the action space 500 is immediately exhausted, energy contained in the medium is lost to the outside. Secondly, when the air transferred into the action space 500 reversely flows to the inlet of the action space 500, it may cause inconvenience to the user. Third, the user may be uncomfortable when the blower is applied only to a portion of the leg.

In the leg care apparatus according to the embodiment, the limitation is greater because the inlet is always opened even while the user uses the leg care apparatus. In addition to the inlet, there may always be a gap between the side module 300 and the main body 100.

Under the background described above, the leg care apparatus according to an embodiment provides a circulation passage through which air in the action space 500 is circulated. This may reduce the air discharged to the outside of the action space 500. The circulation passage refers to a passage through which air to be discharged to the outside of the action space 500 reversely flows into the action space again.

Since the circulation passage is provided, the internal air of the action space 500 and the external air of the action space 500 may be replaced and mixed with each other in various modes according to the needs of the user. For example, the circulation passage through which air is circulated through the action space 500 may be provided. Thus, air outside the action space 500 may be introduced into the action space 500, and air inside the action space 500 is discharged to the outside of the action space 500.

Hereinafter, referring to the drawings, a configuration of the passage of the leg care apparatus according to the embodiment will be described in detail.

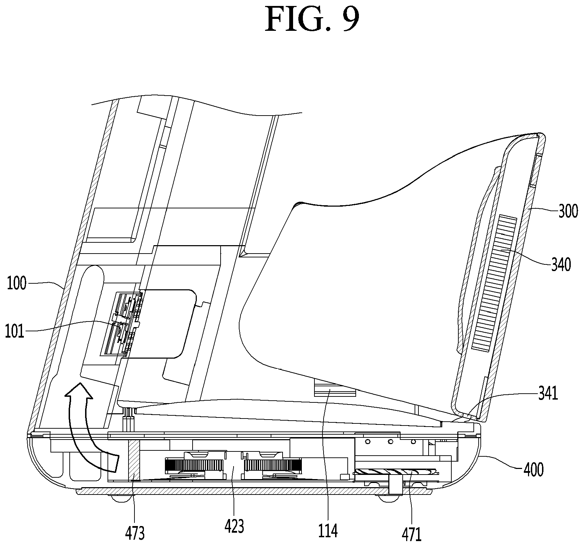

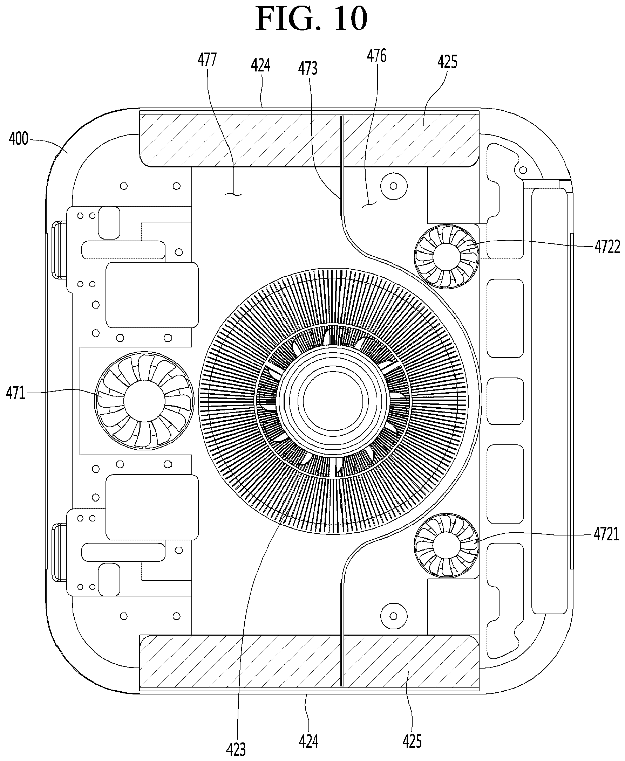

FIG. 9 is a cross-sectional view that cuts vertically an approximately central portion of a lower portion of the leg care apparatus in a front and rear direction, and FIG. 10 is a cross-sectional view that cuts horizontally a lower portion of a bottom module of the leg care apparatus.

Referring to FIGS. 9 and 10, heat exchange fans 340 and 423 may be provided to blow thermal energy exhausted in the thermoelectric modules 330 and 420 to the outside. For example, when the thermoelectric modules 330 and 420 transfer high-temperature heat energy to each of the contact pads 331 and 421, the heat exchange fans 340 and 423 may blow cold air to the outside.

The heat exchange fans may include a side heat exchange fan 340 disposed outside the calf thermoelectric module 330 and a bottom heat exchange fan 423 disposed outside the thermoelectric module 420.

The side heat exchange fan 340 may suction air suctioned from one side of the side module 300 and exhaust the air to the other side of the side module 300. For this, a side through-hole 341 may be provided in an edge of the side module 300. An air current generated by the side heat exchange fan 340 may exchange heat with the calf thermoelectric module 330. The side through-holes 341 may be provided in a lower rear side of the drawing rather than the bottom edge shown in the drawing. This may reduce an action with the action space 500. Alternatively, other places are also possible.

The bottom heat exchange fan 423 may suction air suctioned from one side of left and right sides of the bottom module 400 and exhaust the air suctioned into the other side of the left and right sides of the bottom module 400. For this, the grill 424 and the filter 425 are provided as described above. The air current generated by the bottom heat exchange fan 423 may exchange heat with the foot thermoelectric module 420.

The thermoelectric modules 330 and 420 provide a temperature atmosphere desired by the user into the action space 500. For example, when the user wants cold fomentation, one surface of the thermoelectric modules 330 and 420 provides cold air to each of the corresponding contact pads 331 and 421. In this case, heat is generated on the other surface of each of the thermoelectric modules 330 and 420, and the heat exchange fans 340 and 423 may contact the other surface of each of the thermoelectric modules 330 and 420.

The air used for the heat exchange by the heat exchange fans 340 and 423 may be discharged to the outside because the temperature atmosphere is opposite to the temperature atmosphere desired by the user. That is, it is not desirable to blow hot air to the user who wants the cold fomentation.

The bottom module 400 may further include a circulation air suction fan 471 and an external air suction fan 472 provided in front of the bottom heat exchange fan 423. The external air suction fan 472 may include a first external air suction fan 4721 and a second external air suction fan 4722, which are disposed at both left and right sides of the bottom module 400.

The external air suction fan 472 may suction air from the outside and discharge the air to the blower 101. The circulation air suction fan 471 may suction the internal air of the action space 500 to discharge the air to the blower 101.

When the external air suction fan 472 is operated, the external air may be introduced into the action space 500 through the blower 101. When the circulation air suction fan 471 is operated, the internal air in the action space 500 may be circulated through the blower 101.

When the external air suction fan 472 and the circulation air suction fan 471 are operated together, the external air may be newly introduced into the action space 500, and also, the internal air of the action space 500 may be circulated.

When the external air suction fan 472 is operated, the external air may be newly introduced into the action space 500. Here, the internal air of the action space 500 may be exhausted to the outside. When the external air is suctioned by the external air suction fan 472, air in which foreign substances are filtered by the filter 425 may be introduced.

A state in which only the external air suction fan 472 is operated may be a state in which the internal state of the action space 500 is to be replaced quickly and also may be operations such as initializing the internal state of the action space 500 and cleaning and ventilating the inside of the action space 500 with high humidity. The state in which only the circulation air suction fan 471 is operated may be a foot bath operation in which the foot bath is in progress, i.e., the foot bath is in progress without air leakage to the outside. The state in which the external air suction fan 472 and the circulation air suction fan 471 are operated together may be an operation of gradually changing the internal state of the action space 500. For example, it may be an operation mode for lowering a temperature of the foot bath by injecting in external air.

The operation state adjusted by the operation of the external air suction fan 472 and the circulation air suction fan 471 may be variously configured.

The air flow of the bottom heat exchange fan 423 and the air flow by the external air suction fan 472 have to be blocked from each other. For this, the suction module guide 473 may be further provided on the bottom module 400.

The suction flow guide 473 allows air flow by each fan to be blocked from each other in the bottom module 400. The suction flow guide 473 may divide a space in the bottom module 400 into a suction space 476 through which the external air is suctioned by the external air suction fan 472, and a heat exchange space 477 through which the external air is heat-exchanged by the bottom heat exchange fan 423.

Referring to FIG. 10, in the suction space 476, the external air may be suctioned in a vertical direction to allow the external air to flow upward. Referring to FIG. 10, in the heat exchange space 477, the external air may be suctioned from a lower side to flow upward.

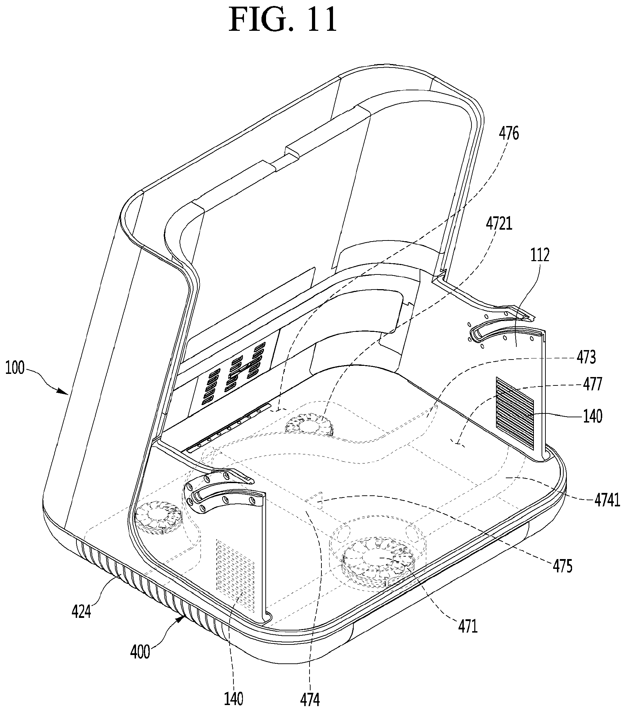

FIG. 11 is a schematic view illustrating the inside of the action space.

Referring to FIG. 11, the main front and rear extension part 112 which block both sides of the action space may have a suction hole 140 for suctioning air in the action space. The suction hole 140 may be provided in each of both the main front and rear extension parts.

The suction hole 140 may be provided in an inner surface of the front portion of the main front and rear extension part 112. Accordingly, a high-energy medium discharged from the blower 101 may not be directly suctioned into the suction hole 140 because the suction hole 140 is spaced apart from the blower 101. Here, the medium may include various media that enhance performance of the foot bath, such as high-temperature mist, heat, and fragrance.

A first suction passage 4741 may be provided to connect the suction hole 140 to the circulation air suction fan 471. The first suction passage 4741 has a pipe structure and may be accommodated in the main body 100 and the bottom module 400. The circulation air suction fan 471 is described as being placed on the bottom module 400, but is not limited thereto. The circulation air suction fan 471 may be accommodated inside the bottom module 400 to simplify an installation space and the passage structure. The circulation air suction fan 471 may suction the medium of the action space from a pair of suction holes 140, i.e., a pair of left and right main front and rear extension parts 112.

A second suction passage 474 connecting a discharge side of the circulation air suction fan 471 to the suction space 476 may be provided. The second suction passage 474 may extend in the front and rear direction of the bottom module 400. The air discharged from the circulation air suction fan 471 may flow into the suction space 476 across the heat exchange space 477. The circulation air discharged into the suction space 476 may be guided to the blower 101 together with the external air discharged from the external air suction fan 472.

As a result, the media in the action space may complete the circulating action of returning out of the action space. The filter or the like may be installed on the circulation path of the medium to filter foreign matter from the circulation air.

When the second suction passage 474 extends to the suction side of the blower 101, the circulation passage may be operated regardless of the action of the external air suction fan 472. For example, the medium inside the action space may complete a complete circulation passage through which the medium is discharged to the outside of the action space regardless of the flow of the external air and then returns again to the action space. Here, the external air may not be mixed.

A damper 475 may be provided in the second suction passage 474. The damper 475 may open or close the second suction passage 474. When the second suction passage 474 is opened, the circulation air suction fan 471 may be operated to circulate air in the action space. When the second suction passage 474 is closed, the circulation air suction fan 471 may be stopped, and the air in the action space may not be circulated.

In this case, the air in the suction space 476 may not flow into the second suction passage 474. Therefore, even if a discharge end of the second suction passage 474 is connected to the discharge side of the external air suction fan 472, the discharged air from the external air suction fan 472 may not reversely flow through the second suction passage 474.

The air flow inside the action space will be described.

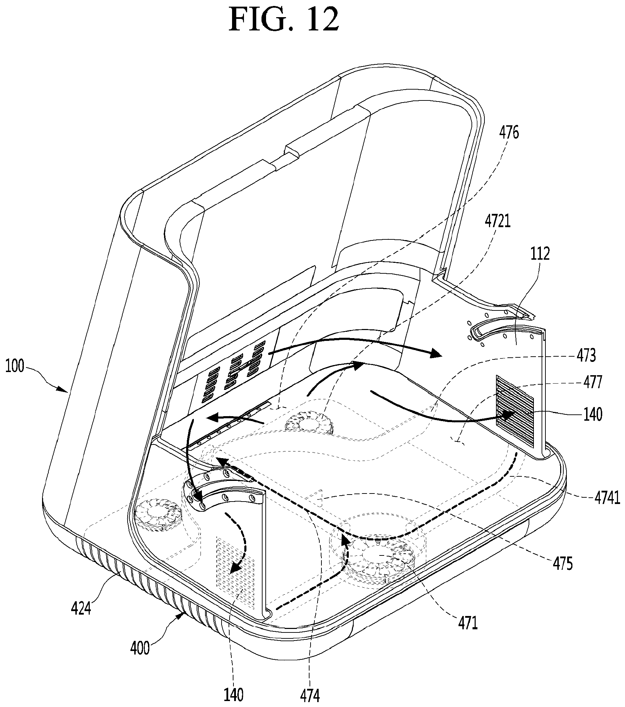

FIG. 12 is a view illustrating the air flow within the action space.

Referring to FIG. 12, the air discharged from the blower 101 may be discharged toward the front side from the rear surface of the action space. The air discharged from the blower 101 may be blown to be spread to the left and right sides while flowing toward the front side. A wind speed and a wind direction of wind discharged from the blower 101 may be adjusted and changed.

However, as a representative case, the air discharged from the blower 101 may be moved forward along the inner surfaces of the pair of main front and rear extension parts 112, and then, the air current at both left and right sides may be moved together at a center.

The suction hole 140 may be provided in a front lower portion of the air current inside the action space, which is a stagnation point of the air current. At the stagnation point, the air in the action space may be exhausted from the action space through the suction hole 140. The stagnation point is a lower corner portion of the action space, and air having a low flow rate and a low temperature may be gathered by density.

The circulation flow of the circulation air discharged from the action space will be described.

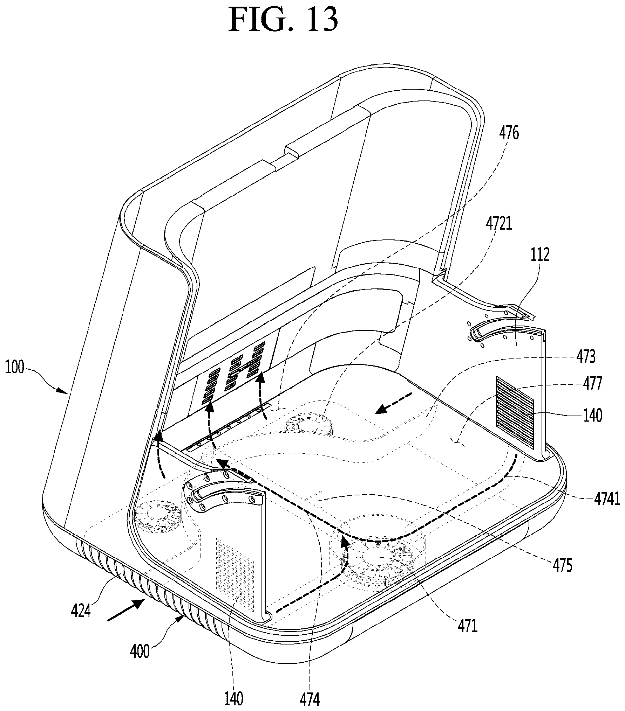

FIG. 13 is a view for explaining a circulation process of the circulation air returning to the action space after being discharged from the action space.

Referring to FIG. 13, the air discharged from the suction hole 140 is introduced into the circulation air suction fan 471 through a first suction channel 4741, which is provided in the side module 300, the main body 100, and the bottom module 400. The suction hole 140 may be provided symmetrical to the left and right sides of the action space. A plurality of suction holes 140 may be provided to suction air from the action space from various places as necessary.

The circulation air suction fan 471 suctions air in the action space symmetrically through the suction hole 140 provided symmetrically. Thus, it may contribute to obtain the same foot bath effect on both feet.

The air discharged from the circulation air suction fan 471 is guided to the suction space 476 through the second suction passage 474. The second suction passage 474 passes through the heat exchange space 477. The internal flow air of the second suction passage 474 may not be mixed with the internal air of the heat exchange space 477.

The external air suction fan 472 may forcibly suction external air into the suction space 476 and blow air to the blower 101. A discharge end of the second suction passage 474 may be connected to a suction end of the external air suction fan 472 or may be connected to the discharge end.

When the discharge end of the second suction passage 474 is connected to the suction end of the external air suction fan 472, the external air and the circulation air may be sufficiently mixed to be blown by the blower 101. On the contrary, when the discharge end of the second suction passage 474 is connected to the discharge end of the external air suction fan 472, a flow loss may be reduced because it is unnecessary to pass through the external air suction fan 472 when only the circulation air is blown without the external air.

As described above, in the action space 500 according to an embodiment, the medium for the foot bath, which contains the air inside the action space, is discharged to the outside through various intervals in addition to the inlet. For this reason, there is a concern of energy loss, user inconvenience, and maintenance cost increase. On the contrary, there is a case in which the air inside the action space has to be actively discharged for cleaning the action space, releasing moisture, and changing the atmosphere of the action space.

Hereinafter, a case of operating the external air suction fan 472 and the circulation air suction fan 471 according to each case will be described in more detail.

The external air suction fan 472 may blow air outside the action space into the action space. The circulation air suction fan 471 discharges the air inside the action space to the outside of the action space and then blows the air into the action space again.

The circulation air suction fan 471 may prevent the air discharged from the blower 101 from being discharged to the outside. As an extreme example, if a flow rate per unit time of the blower 101 and a flow rate per unit time of the circulation air suction fan 471 are the same, there may be no air or medium discharged to the outside of the action space, i.e., the indoor space.

Each case will be described.

First, when only the external air suction fan 472 is operated, the external air continues to flow into the action space. Here, the air in the action space may be discharged to the outside of the action space. This mode may be used in a case of drying an inner wall of the action space.

Second, when only the circulation air suction fan 471 is operated, the external air does not flow into the action space newly. Instead, the air in the action space may be circulated to return to the inside of the action space again. In this case, large amount of energy may be sufficiently contained inside the action space, and the foot bath may be performed for a long time. This mode has an advantage of high energy efficiency.

Third, when an air volume of the external air suction fan 472 is less than that of the circulation air suction fan 471, the external air flows into the action space newly, but an amount of external air may be small. In this case, an amount of air discharged to the outside may be compensated through the interval between the inlet of the action space and the components. In other words, in the leg care apparatus according to an embodiment, since the action space is not provided in a closed type, the air inside the action space may be exhausted by a predetermined amount to the outside. As described above, it is preferable to allow a certain amount of external air to flow into the action space so as to balance an amount of air exhausted to the outside of the action space.

Fourth, when an air volume of the circulation air suction fan 471 is less than that of the external air suction fan 472, the newly introduced external air may be greater than that of air circulating through the action space. In this case, the air inside the action space may be discharged to the outside by a predetermined amount, or the air containing the medium may be applied to the human body portion adjacent to the inlet side, for example, a thigh or a knee.

As seen from the above description, the leg care apparatus according to the embodiment, in a normal state, the external air suction fan 472 and the circulation air suction fan 471 may be driven together. In this case, a circulation air suction amount may be greater than the air volume of the external air suction fan 472. In this driving state, the external air may newly flow to the action space in an amount sufficient to compensate for the air naturally exhausted from the action space to the outside.

To allow the various operating states to be individually performed, it is preferable that any one of the external air suction fan 472 and the circulation air suction fan 471 be configured in which a rotation rate of the respective fans is controlled.

The leg care apparatus according to an embodiment allows a large portion of the leg, which includes the knee and thigh, to be placed inside the action space. The user's leg may be aligned with both feet from side to side. The user may be seated, and each leg may be provided lengthily in a vertical direction.

Air containing the medium necessary for the foot bath may be added to each of the left and right legs of the user placed in the action space. Since the user's leg is provided lengthily in the vertical direction, it is preferable to allow the medium to affect the whole leg correspondingly. To allow the medium to be applied to each of the left and right legs, the blower may be provided with a pair so as to correspond to each leg.

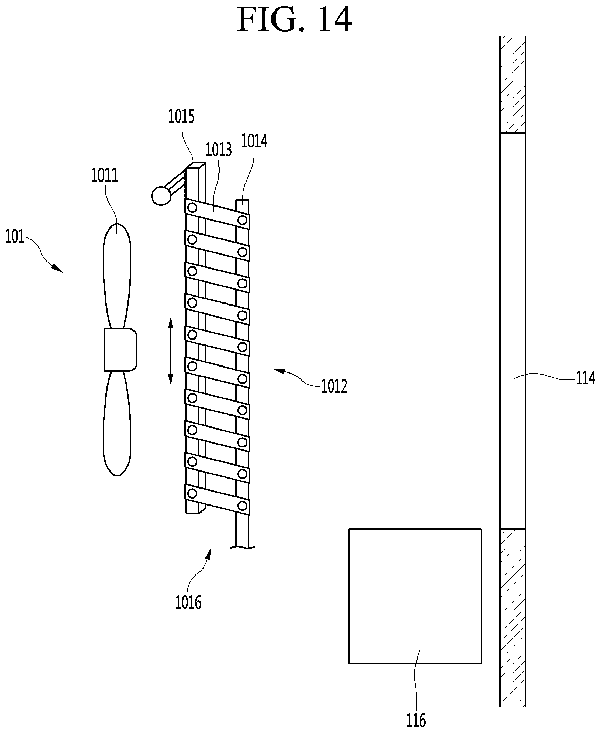

FIG. 14 is a configuration view for explaining an example of the blower.

Referring to FIG. 14, the blower 101 includes a fan 1011 and a wind direction controller 1016 placed at the discharge side of the fan 1011.

The atomizer 116 may be provided in front of the blower 101 to allow air containing the mist to be blown into the action space. Alternatively, fragrance may be provided through a fragrance kit.

The blower 101 and the atomizer 116 may be fixed at a position near a rear opening 114 of the main body 100. The blower 101 may blow the medium through the rear opening 114 to supply the medium to the action space.

The wind direction controller 1016 may include a wind direction control stand 1013 controlling a wind direction, a fixing stand 1014 on which one side of the wind direction control stand 1013 is rotatably hinge-installed, and a movable stand 1015 on which the other side of the wind direction control stand 1013 is hinge-rotatable and which is installed to be vertically translated. The movable stand 1015 is engaged with a motor to allow the movable stand 1015 to be translated vertically.

Since the wind direction controller 1016 is operated, air discharged forward from the blower 101 may be controlled in wind direction vertically, and thus be discharged to the inside of the action space. Since the discharged wind direction of the blower 101 is controlled vertically, the air containing the medium may be affected to all portions of the user's feet. As a result, a smooth foot bath function may be performed.

The wind direction controller 1016 is shown to control a direction of the air of the blower vertically. In addition, it is also possible to further provide a configuration to control the direction of the air in a left and right direction by disposing the wind direction control stand, the fixing stand, and the movable stand in the left and right direction.

The leg care apparatus according to the embodiment may be moist in the action space, the waste attached to the foot may emit an odor due to decomposition. This odor may lead to situations in which the leg care apparatus needs to be ventilated or cleaned as a whole.

Hereinafter, an embodiment that copes with the odor described above is proposed.

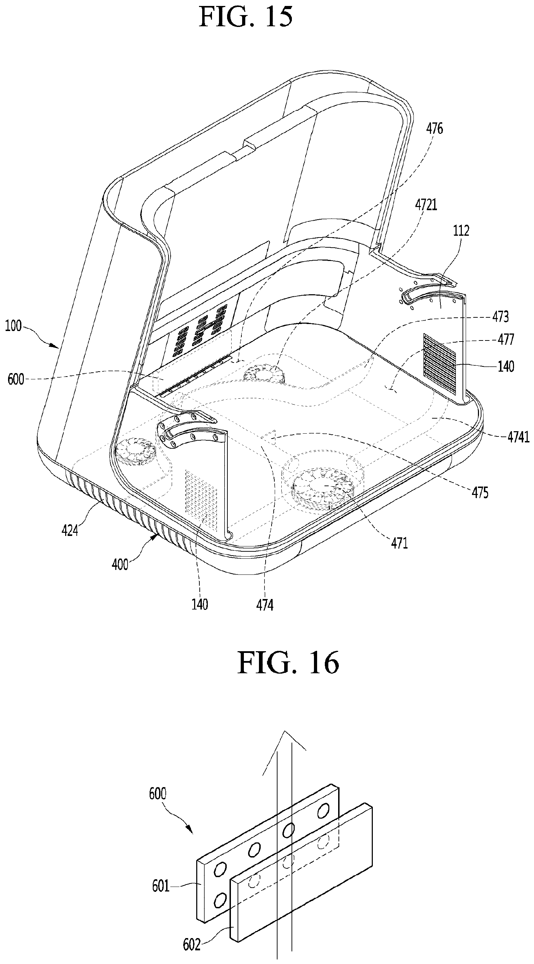

FIG. 15 is a configuration of a leg care apparatus according to another embodiment, and only portions that are characteristically different from those of the embodiment shown in FIG 11 may be illustrated, and different portions will be omitted.

Referring to FIG. 15, an odor removing device 600 may be further provided on a passage guided from the suction space 476 to the blower 101. Although provided on a directly adjacent downstream side of the blower 101, since a strong flow rate of the blower 101 may interfere with the odor removing action, it is not preferable. However, the installation is not impossible. The odor removing device 600 may be installed in a space in which a flow rate is slow in a large space.

The odor removing device 600 may remove odor particles from air discharged from the action space so as to be circulated. The odor removing device 600 may remove the odor from the air discharged from the action space and the air is supplied again, thereby maintaining the action space to be more clean.

Since the odor removing device 600 is provided downstream of the external air suction fan 472, odor particles of air introduced from the outside may be removed. Accordingly, it is possible to remove not only the air inside the action space but also the odor of the indoor space in which the leg care apparatus is placed.

With aging, humans secrete odor particles called nonenal, and the nonenal is recognized as a unique ingredient from the elderly. The odor in the indoor space of the elderly is mainly due to the nonenal. The leg care apparatus according to an embodiment may not only help the healthy life of the elderly enjoying foot baths, but also may help to live a more comfortable and clean life by removing nonenal particles from the elderly living space.

If it is not necessary to remove the odor particles to the outside of the action space, the odor removing device may be provided at different area. For example, the odor removing device may be disposed at various positions such as any inner surface facing the air in the action space, at least one of upstream and downstream of the circulation air suction fan 471, and the like.

FIG. 16 is a view illustrating an example of the odor removing device.

The odor removing device according to the embodiment may include a catalyst member 602 in which a photocatalyst that is exemplified by titanium oxide (TiO₂) is exposed to air and an optical array 601 that emits light to the catalyst member 602 so that the catalyst member 602 increases in activity.

The optical array is a member for allowing the activity of the catalyst to increase, and also, a light emitting element having high ultraviolet intensity may be used as the optical array. The ultraviolet rays are very effective for the sterilization of pathogenic bacteria.

The photocatalytic reaction will be described.

When light having a wavelength of about 400 nm or less is close to a surface of the photocatalyst, oxygen and water molecules in the air are broken to generate photoelectrons and ozone. The ozone is effective in sterilization.

In addition, OH radicals generated on a surface of titanium oxide may be utilized to decompose organic matter, thereby removing odor particles.

Specifically, titanium oxide generates holes by light having an ultraviolet band. Water adsorbed on the surface of the hole and the titanium oxide reacts to produce OH radicals, and in the case of oxygen existing in water, reacts with electrons to generate 02- radicals, thereby generating more OH radicals. The OH radical may decompose organic materials on the surface of titanium oxide. This is called a photocatalytic reaction.

Semiconductor materials used as photocatalysts include TiO₂, SiO₂, ZnO, and WO₃. The reason why titanium oxide (TiO₂) is most popular is because it is highly active, inexpensive, harmless to the human body, and has chemical stability.

As described above, the optical array 601 and the catalyst member 602 may serve to remove odor particles and sterilize.

On the other hand, with the operation of the odor removing device 600, the odor removing device provided in the fragrance kit 103 may be also operated together. In addition, aroma scent provided from the perfume of the fragrance kit 130 may further enhance the user's satisfaction.

The catalyst member 602 is a porous member having a large surface area as much as possible and may have high efficiency of collecting and removing the odor particles.

In this embodiment, the indoor environment may be maintained to be more clean by removing the odors such as sweat and nonenal derived from the human body.

Another embodiment included in the spirit of the present disclosure is further described.

One of the upper module and the side module may not be provided. Thus, only one of the upper module and the side module may be opened so that the user inserts a leg into an action space. In this case, there is a limitation that the user's inconvenience slightly increases, but the operation and action of the leg care apparatus is not impossible. Nevertheless, the embodiment in which both the upper module and the side module are provided is most preferable for the convenience of the user.

One of the foot contact pad and the calf contact pad may not be provided. Thus, even if only one contact pad is used, heat and cold air may be transferred to the legs by the other heat transfer part. In this case, there is a limitation that the user's inconvenience slightly increases, but the operation and action of the leg care apparatus is not impossible. Nevertheless, the embodiment in which both the leg contact pad and the calf contact pad are provided is most preferable for the convenience of the user.

The circulation air suction fan and the external air suction fan have no problem in operation of the leg care apparatus even if only one is provided. However, when both types of suction fans are provided, both the action of the external air and the action of the circulation air may be derived, and thus, the user's satisfaction may be enhanced.

The circulation air suction fan may not be provided. For example, the air in the action space may be discharged at an interval between the inlet 510 and each module.

For another example, the suction hole 140 may be connected to the inlet-side of the blower 101 by a predetermined passage, and the circulation passage may be provided by a negative pressure of the blower 101. In this case, the circulation passage may not be smooth. However, even in this case, the operation of the leg care apparatus is not impossible, and the air flow inside the action space may be smoothed by a method of distributing the positions of the suction holes.

According to the present disclosure, even the elderly may conveniently care the leg thereof by using the foot bath, in particular, since the handling difficulties caused when using a lot of water is eliminated, even people with limited mobility may use the leg care apparatus conveniently.

In particular, the elderly who suffer from orthopedic diseases may reduce the pain without relying on the drug and may perform the minimum movement that is necessary for the operation of the apparatus.

According to the embodiment, the user may adjust the size and the like of the leg care apparatus to be suitable for his/her own body and conveniently operate the leg care apparatus.

According to the embodiment, since the leg care apparatus is safely used even in the high humidity environment, the risks of the burn and the electric shock may be reduced.

According to the embodiment, the use may conveniently move the leg care apparatus, and the leg care apparatus may be conveniently used in the narrow indoor space due to the compact size thereof.

According to the embodiment, the leg care apparatus may include the firm frame so as to be used for a long time without being damaged.

According to the embodiment, since the heat atmosphere of the heating element is transmitted to the feet in the various manners such as conduction, convection, and radiation, the foot bath effect may be improved, and the user's satisfaction may increase.

According to the embodiment, since the hot and cold air are directly transferred to the portion at which the hot and cold air are required, the more improved foot bath effect may be obtained, and the energy consumption may be saved.

According to the embodiment, the foot bath may be performed through all manners of conduction, convection, and radiation.

According to the embodiment, the odor generated in the action space may be removed so that the leg care apparatus is more cleanly used.

## Claims

1. A leg care apparatus comprising:
a main body (100) configured to provide an action space (500) to accommodate a leg;
a bottom module (400) disposed at a bottom surface of the main body (100) and in which components for a foot bath are accommodated; and
a circulation air suction fan (471) configured to exhaust air within the action space (500) to an outside of the action space (500) and blow the air exhausted to the outside of the action space (500) back into the action space (500).

2. The leg care apparatus according to claim 1, further comprising an external air suction fan (472) configured to introduce external air outside the action space (500) into the action space (500).

3. The leg care apparatus according to claim 2, further comprising a foot thermoelectric module (420) disposed at the bottom module (400) and having one surface to correspond with one surface within the action space (500).

4. The leg care apparatus according to claim 3, further comprising a bottom heat exchange fan (423) disposed at the bottom module (400) to heat-exchange with another surface of the foot thermoelectric module (420).

5. The leg care apparatus according to claim 4, further comprising a suction passage guide (473) disposed inside the bottom module (400) that divides an inner space of the bottom module (400) into a suction space through which the external air flows by discharge of the external air suction fan (472) and a heat exchange space through which the external air flows by discharge of the bottom heat exchange fan (423).

6. The leg care apparatus according to claim 2, wherein the external air discharged by the external air suction fan (472) and the air discharged by the circulation air suction fan (471) are introduced into the action space (500).

7. The leg care apparatus according to claim 2, wherein the external air suction fan (472) suctions the external air from both sides of the bottom module (400).

8. The leg care apparatus according to any one of claims 1 to 7, further comprising a blower (101) corresponding to a rear surface of the action space (500),
wherein the blower (101) comprises:
a fan (1011); and
a wind direction controller (1016) disposed at a discharge-side of the fan (1011).

9. The leg care apparatus according to claim 8, wherein the wind direction controller (1016) is configured to control a wind direction vertically.

10. The leg care apparatus according to claim 8, wherein the blower (101) comprises a left blower and a right blower.

11. The leg care apparatus according to any one of claims 1 to 10, further comprising an odor removing device (600) configured to remove odor particles from the air discharged from the circulation air suction fan (471).

12. The leg care apparatus according to claim 11, insofar as depending on claim 8, wherein the odor removing device (600) is disposed in a passage between a discharge-side of the circulation air suction fan (471) and a suction-side of the blower (101).

13. The leg care apparatus according to claim 11 or 12, wherein the odor removing device (600) comprises:
a catalyst member (602); and
a light emitting element (601) configured to emit light to the catalyst member (602).

14. The leg care apparatus according to any one of claims 1 to 13, further comprising a fragrance kit (130) configured to provide fragrance to a passage of a discharge-side of the circulation air suction fan (471).

15. The leg care apparatus according to any one of claims 2 to 14, insofar as depending on claim 2, wherein a wind volume flowing by the circulation air suction fan (471) and a wind volume flowing by the external air suction fan (472) are different from each other.
